# EUROPEAN PATENT SPECIFICATION

(11) **EP 0 973 790 B1**
(45) Date of publication and mention of the grant of the patent: **04.06.2003**
(21) Application number: 98915224.4
(22) Date of filing: 02.04.1998
(51) Int. Cl.: C07J 1/00, A61K 31/565, C07J 31/00, C07J 17/00, C07J 21/00, A61K 31/58

(54) **ESTRA-5(10),7-DIENES WITH ESTROGENIC ACTIVITY**
ESTRA-5(10),7-DIENE MIT ÖSTROGENER AKTIVITÄT
OESTRA-5(10),7-DIENES AVEC ACTIVITE OESTROGENIQUE

(30) Priority: 07.04.1997 US 835336; 02.05.1997 US 850570
(43) Date of publication of application: 26.01.2000
(73) Proprietor: Wyeth, Madison, New Jersey 07940-0874 (US)
(72) Inventor: KONG, Fangming, Riverale, NJ 07675 (US); McDONALD, Leonard, Alexander, Mountainside, NJ 07092 (US); KAGAN, Michael, Z., Plainsboro, NJ 08536 (US); SHAH, Syed, Muzafar, East Hanover, NJ 07936 (US); RAVEENDRANATH, Panolil, Monroe, NY 10950 (US); COLLINS, Mark, Andrew, Norristown, PA 19401 (US)
(74) Representative: Connelly, Michael John
(86) International application number: US9806461
(87) International publication number: WO98045315

(56) References cited:
- DE-B- 1 093 360
- US-A- 2 930 805
- US-A- 3 340 278
- JUNGHANS K ET AL: "Preparative organic electrochemistry. IV. Solvent effects in the electrolytic reduction of steroids with aromatic A and B rings" CHEMISCHE BERICHTE., vol. 112, no. 7, 1979, WEINHEIM DE, pages 2631-2639, XP002070450
- DATABASE WPI Section Ch, Derwent Publications Ltd., London, GB; Class B01, AN 71-77128S XP002070452 & JP 46 040 939 B (TAKEDA CHEM IND LTD)
- CHEMICAL ABSTRACTS, vol. 076, no. 7, 14 February 1972 Columbus, Ohio, US; abstract no. 034484, HIRAGA K ET AL: ".DELTA.7-Steroids" XP002070451 & JP 46 040 939 - (TAKEDA CHEMICAL INDUSTRIES, LTD.) 3 December 1971

## Description

### BACKGROUND OF THE INVENTION

The use of naturally occurring estrogenic compositions of substantial purity and low toxicity such as PREMARIN (conjugated equine estrogens) has become a preferred medical treatment for alleviating the symptoms of menopausal syndrome, osteoporosis/osteopenia in estrogen deficient women and in other hormone related disorders. PREMARIN is a registered trade mark. The estrogenic components of the naturally occurring estrogenic compositions have been generally identified as sulfate esters of estrone, equilin, equilenin, 17β-estradiol, dihydroequilenin and 17β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, estra-5(10),7-dien-3β-ol-17-one 3-sulfate ester sodium salt is a minor component of PREMARIN (conjugated equine estrogens). U.S. Patent 2,930,805 discloses the preparation of 3,17β-dihydroxy-5(10),7-estradienes, and their use as antiestrogens. U.S. Patent 3,340,278 discloses the preparation of 5(10),7-estradien-3,17-dione, 3,17β-dihydroxy-5(10),7-estradiene, and 17β-hydroxy-5(10),7-estradien-3-one, which are useful as intermediates in the preparation of equilin. Junghans et al., Chem. Ber. 112, 2631-2639 (1979) discloses the preparation of 17-ethylenedioxy-5(10),7-estradien-3α-ol and 17-ethylenedioxy-5(10),7-estradien-3β-ol by electrolysis of 17-ethylenedioxy-5(10),6,8-estratrien-3α-ol and 17-ethylenedioxy-5(10),6,8-estratrien-3β-ol respectively in liquid methylamine.

### DESCRIPTION OF THE INVENTION

In accordance with this invention, there are provided estra-5(10),7-dien-3β-ol-17-one or a pharmaceutically acceptable salt of its 3-sulfate ester, estra-5(10),7-dien-3β-ol-17-one 3-glucuronide or a pharmaceutically acceptable salt thereof, estra-5(10),7-dien-3α-ol-17-one or a pharmaceutically acceptable salt of its 3-sulfate ester, and estra-5(10),7-dien-3α-ol-17-one 3-glucuronide or a pharmaceutically acceptable salt thereof. This invention also provides a compound which is a 3-alkali metal salt of estra-5(10),7-dien-3β-ol-17-one or estra-5(10),7-dien-3α-ol-17-one. These are all collectively referred to as the compounds of this invention. The structures of estra-5(10),7-dien 3β-ol-17-one and estra-5(10),7-dien-3α-ol-17-one are shown below as compounds **5B** and **5A**, respectively.

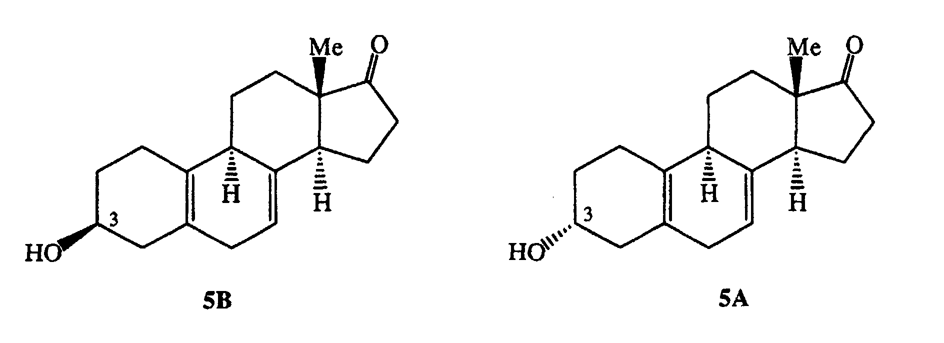

Pharmaceutically acceptable salts of estra-5(10),7-dien-3β-ol-17-one 3-sulfate ester, estra-5(10),7-dien-3β-ol-17-one 3-glucuronide, estra-5(10),7-dien-3α-ol-17-one 3-sulfate ester, or estra-5(10),7-dien-3α-ol-17-one 3-glucuronide include, but are not limited to, the alkali metal salts, alkaline earth metal salts, ammonium salts, alkylammonium salts containing 1-6 carbon atoms or dialkylammonium salts containing 1-6 carbon atoms in each alkyl group, and trialkylammonium salts containing 1-6 carbon atoms in each alkyl group. The alkali metal of the 3-alkali metal salts of estra-5(10),7-dien-3β-ol-17-one or estra-5(10),7-dien-3α-ol-17-one may be lithium, sodium, or potassium.

As estra-5(10),7-dien-3β-ol-17-one 3-sulfate ester sodium salt is a minor component of PREMARIN (conjugated equine estrogens), this invention also provides estra-5(10),7-dien-3β-ol-17-one 3-sulfate sodium salt in greater than one percent purity.

This invention also provides a compound consisting essentially of estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof; and a compound consisting essentially of estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

As used in accordance with this invention, treating covers treatment of an existing condition, ameliorating the condition, or providing palliation of the condition and inhibiting includes inhibiting or preventing the progress or development of the condition.

The compounds of this invention may be prepared by a process which comprises
(a) deprotecting a 17- protected 3-hydroxyestra-5(10),7-dien-17-one, for example, 17-ethylenedioxyestra-5(10),7-dien-3-ol or a 3- protected 3-hydroxyestra-5(10),7-dien-17-one, for example, 3-(tertiary butyldiphenylsilyl)oxyestra-5(10),7-dien-17-one and recovering 3α-hydroxyestra-5(10),7-dien-17-one or 3β-hydroxyestra-5(10),7-dien-17-one; or
(b) subjecting 3β-hydroxyestra-5(10),7-dien-17-one to a Mitsunobu reaction and recovering 3α-hydroxyestra-5(10),7-dien-17-one; or
(c) converting 3α-hydroxyestra-5(10),7-dien-17-one into a 3-sulfate ester thereof by reaction with a sulfur trioxide-ammonia, -alkylamine, -dialkylamine or -trialkylamine reagent such as sulfur trioxide-triethylamine or sulfur trioxide -pyridine to form an ammonium, alkylammonium, dialkylammonium, trialkylammonium or pyridinium 3-sulfate salt and, if desired, converting the 3-sulfate salt into another 3-sulfate salt by exchange of cations, e.g. by forming a metal salt by treatment with a metal hydroxide solution; or
(d) converting 3β-hydroxyestra-5(10),7-dien-17-one into a 3-sulfate ester thereof by reaction with a sulfur trioxide-ammonia, -alkylamine, -dialkylamine or -trialkylamine reagent such as sulfur trioxide-triethylamine or sulfur trioxide -pyridine to form an ammonium, alkylammonium, dialkylammonium, trialkylammonium or pyridinium 3-sulfate salt and, if desired, converting the 3-sulfate salt into another 3-sulfate salt by exchange of cations, e.g. by forming a metal salt by treatment with a metal hydroxide solution; or
(e) converting 3α-hydroxyestra-5(10),7-dien-17-one into its 3-glucuronide or sodium salt thereof; or
(f) converting 3β-hydroxyestra-5(10),7-dien-17-one into its 3-glucuronide or sodium salt thereof; or
(g) converting 3α-hydroxyestra-5(10),7-dien-17-one 3-glucuronide into a pharmaceutically acceptable salt thereof by neutralisation with a base; or
(h) converting 3β-hydroxyestra-5(10),7-dien-17-one 3-glucuronide into a pharmaceutically acceptable salt thereof by neutralisation with a base; or
(i) converting 3α-hydroxyestra-5(10),7-dien-17-one into an alkali metal salt thereof by reaction with an alkali metal-containing base; or
(j) converting 3β-hydroxyestra-5(10),7-dien-17-one into an alkali metal salt thereof by reaction with an alkali metal-containing base.

The compounds of this invention can be prepared from readily available starting materials. For example, the preparation of estra-5(10),7-dien-3β-ol-17-one **(5B)**, estra-5(10),7-dien-3α-ol-17-one **(5A)**, estra-5(10),7-dien-3α-ol- 17-one 3-sulfate ester sodium salt **(7)**, and estra-5(10),7-dien-3β-ol-17-one 3-sulfate ester triethylammonium salt **(15)** are shown in Schemes I and II starting from equilin methyl ether (U.S. Patent 3,644,439) and 17β-hydroxyestra-5(10),7-dien-3-one **(8)** (U.S. Patent 2,930,805). Estra-5(10),7-dien-3β-ol-17-one 3-glucuronide sodium salt **(16)** and estra-5(10),7-dien-3α-ol-17-one 3-glucuronide sodium salt **(17)** can be prepared according to Scheme m.

As shown in Scheme I, the 17-ethylenedioxy derivative of equilin methylether **1** is converted to the triene **2** utilizing a Birch reduction with lithium in liquid ammonia. Mild oxalic acid treatment allows the selective hydrolysis of the enol ether to provide the ketone **3**. Sequential reduction of the 3-ketone (lithium aluminum hydride); and deprotection of the 17-ketone (p-tolunesulfonic acid) affords the products **5A** and **5B** of the invention as a mixture in which the 3α-isomer **5A** predominates (4:1 ratio). Separation can be realized directly by preparative high pressure liquid chromatography. Alternatively, inversion of configuration at C-3 to a mixture in which the 3β-isomer predominates can be achieved by a Mitsunobu reaction. The highly crystalline 3-(3,5-dinitro)benzoates, products of the Mitsunobu inversion, are readily separable by column chromatography. After hydrolysis, **5B** was converted to its 3β-sulfate 7 with triethylamine:sufur trioxide reagent.

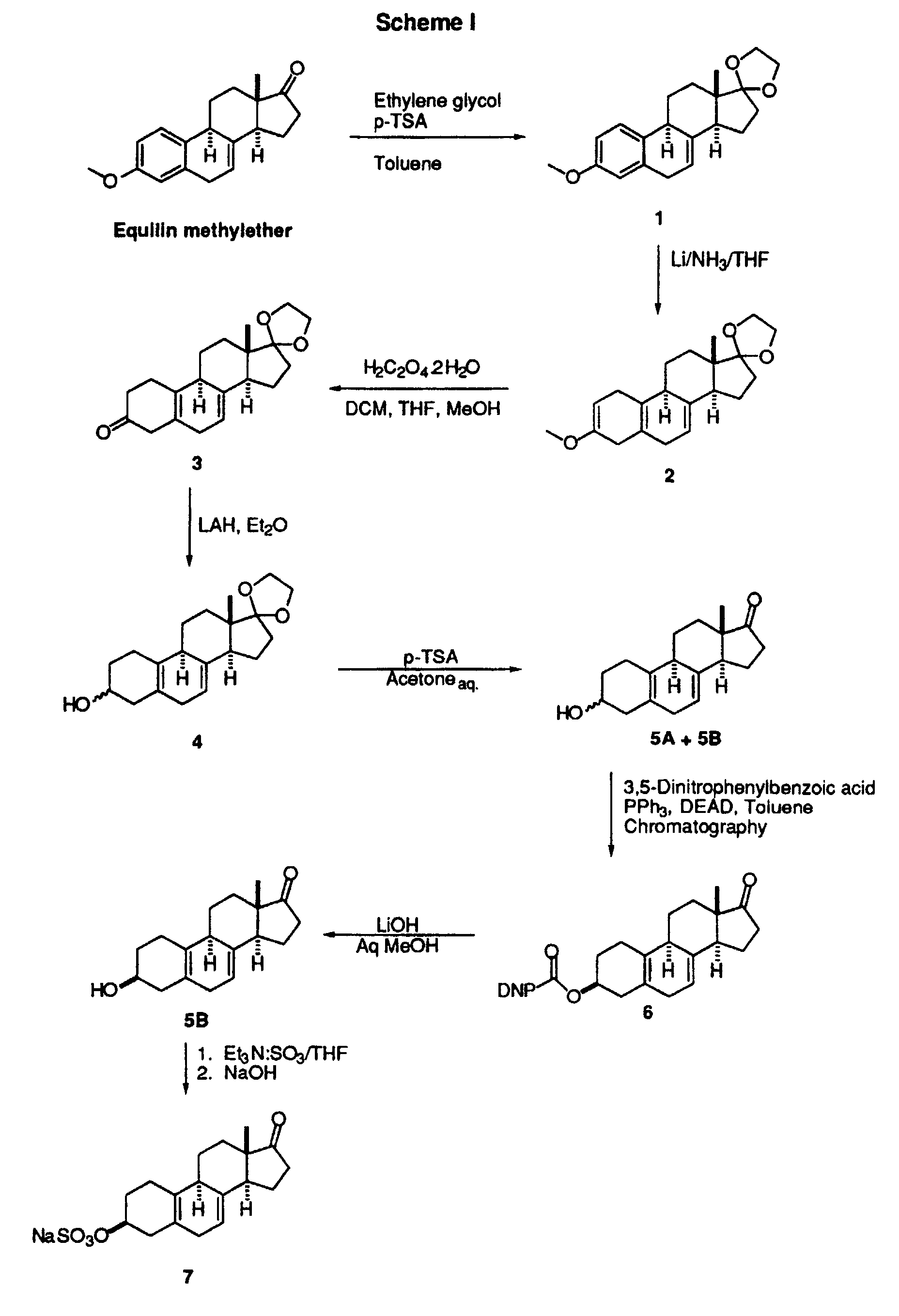

Scheme II outlines the conversion of 17β-hydroxyestra-5(10),7-dien-3-one **(8)** to a mixture of **5A** and **5B** (6.6:1 ratio) by a reduction-oxidation sequence requiring a differential protection-deprotection sequence for the C-3 and C-17 functionalities. Reduction at C-3 utilized lithium tri-tertbutoxyaluminum hydride, a Swern-type oxidation provided the C-17 ketone. Separation of **5A** and **5B** was achieved by HPLC. The conversion of **5A** to the 3α-sulfate **15** with pyridine:sufur trioxide reagent is exemplified. The alkali metal salts of **5A** and **5B** can be prepared by treatment of the respective alcohol with an alkali metal hydride, such as sodium hydride, in a nonaqueous solvent such as THF or DMF. The alkali metal salts of **5A** and **5B** are useful as intermediates in the preparation of the sulfate esters (via amine:sulfur trioxide treatment) of **5A** and **5B**, and are also useful as estrogenic compounds.

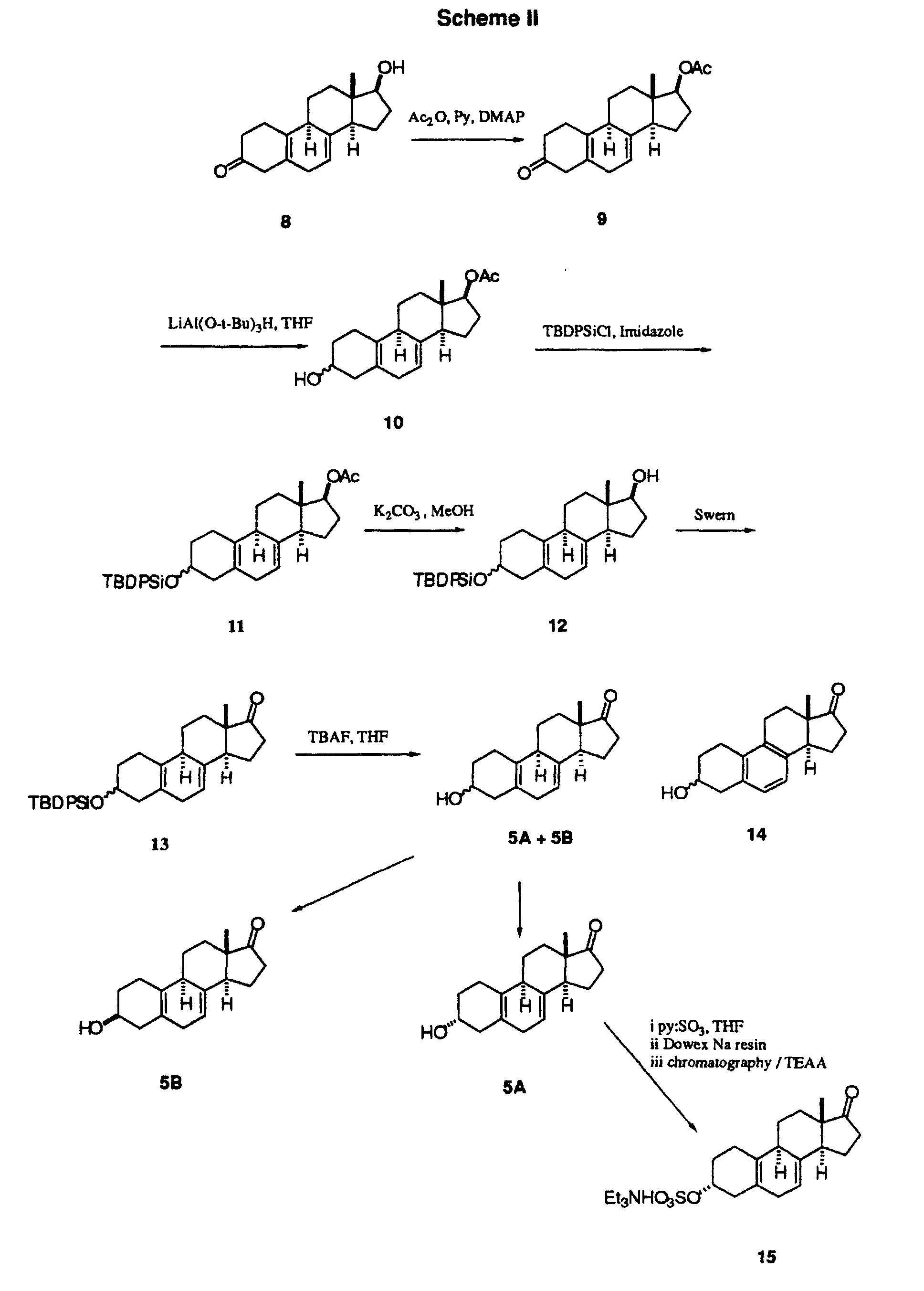

Scheme III shows the preparation of estra-5(10),7-dien-3β-ol-17-one 3-glucuronide sodium salt **(16)** and estra-5(10),7-dien-3α-ol-17-one 3-glucuronide sodium salt **(17)** from estra-5(10),7-dien-3β-ol-17-one **(5B)** and estra-5(10),7-dien-3α-ol-17-one **(5A)**, respectively. The sodium glucuronides **(16)** and **(17)** can be treated with mild acid to provide the respective 3-glucuronides.

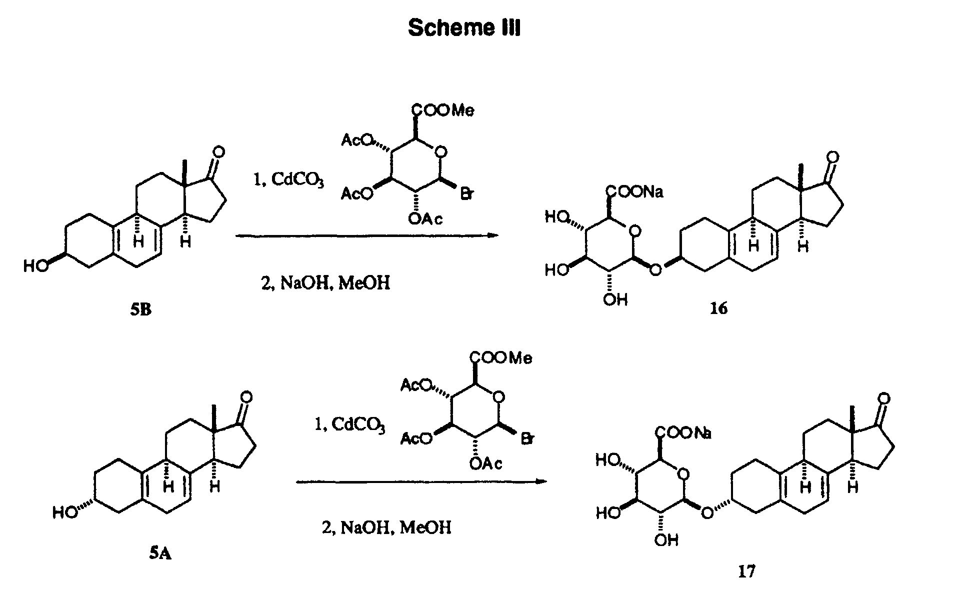

The compounds of this invention are estrogenic, as shown in the in vitro and in vivo standard pharmacological test procedures described below in which compounds estra-5(10),7-dien-3β-ol-17-one **(5B)** and estra-5(10),7-dien-3α-ol-17-one **(5A)** were evaluated as representative compounds of this invention.

### Estrogen Receptor Binding

An initial evaluation examined the competitive binding properties of **5B** and **5A** to the human estrogen receptor (hER-α) prepared as a soluble cell extract (cytosol). In this standard pharmacological test procedure, **5B** and **5A** demonstrated no specific binding activity. However, when estrogen receptor binding was analyzed using a whole cell test procedure, specific binding was clearly demonstrated. This test procedure indicated an IC₅₀ of 1.5 x 10⁻⁷ M or an estimated Kᵢ of 150nM for **5B**. Similarly, **5A** demonstrated an IC₅₀ of 2 x 10⁻⁷ M. This would be compared with a Kᵢ for estrone, equilin and equilenen of 51, 67 and 375nM, respectively.

### In Vitro Co-Transfection Test Procedure

In this standard pharmacological test procedure, hER-α over-expressed in Chinese hamster ovary (CHO) cells infected with adeno-2x-ERE-tk-luciferase, an estrogen responsive reporter gene construct, cells were exposed to varying concentrations (10⁻¹²-10⁻⁵M) of **5B** or **5A** for 24 hours. Cells were also exposed to 17β-estradiol at 10⁻⁹ M. Following the 24-hour treatment, cells were lysed and cell extracts assayed for luciferase activity. The results provided that **5B** had an EC₅₀ of approximately 29nM and **5A** of 43nM. Using a similar test procedure, previous data indicate a 5.6nM EC₅₀ for estrone.

### In Vivo Uterotropic Activity

Immature rats were treated with varying doses of **5B** or **5A** for three days (S.C.) as well as additional groups (n=6) of rats treated with 0.5 µg ethinyl estradiol and vehicle as positive and negative controls, respectively. The results of this standard pharmacological test procedure are presented in the table below.

| **Treatment (n=6/group)** | **Uterine Weight (mg) ± SD** | **Significant Differences from Vehicle** |
|---|---|---|
| Vehicle | 25.4 ± 4.4 | -- |
| Ethinyl Estradiol (0.5 µg) | 101.2 ±2.8 | p <.001 |
| **5B** (500 µg) | 82.6 ± 8.6 | p <.001 |
| **5B** (100 µg) | 79.7 ± 5.8 | p <.001 |
| **5B** (10 µg) | 43.5 ± 6.9 | p <.001 |
| **5B** (1 µg) | 31.7 ± 6.6 | p = .11 |
| **5B** (0.1 µg) | 34.1 ± 6.2 | p = .03 |
| **5A** (500 µg) | 78.9 ± 13.6 | p <.001 |
| **5A** (100 µg) | 66.5 ± 4.7 | p <.001 |
| **5A** (10 µg) | 33.3 ± 4.4 | p = .048 |
| **5A** (1 µg) | 28.7 ± 2.7 | p = .40 |

The results obtained demonstrate significant uterine stimulation compared with vehicle at almost all doses. At doses above 10 µg/rat, the difference from vehicle was significant at a p-value less than 0.001 for both **5B** and **5A**. The estimated EC₅₀ from this test procedure would be approximately 30 µg/rat or 0.6 mg/kg for **5B** and somewhat lower for **5A**. The EC₅₀ for estrone has been estimated to be 0.2 mg/kg in similar test procedures. Thus, the data from this *in vivo* standard pharmacological test procedure data demonstrate that **5B** and **5A** have significant estrogenic activity.

Based on the results of these standard pharmacological test procedures using representative compounds of this invention, estra-5(10),7-dien-3β-ol-17-one or a pharmaceutically acceptable salt of its 3-sulfate ester, estra-5(10),7-dien-3β-ol-17-one 3-glucuronide or a pharmaceutically acceptable salt thereof, estra-5(10),7-dien-3α-ol-17-one or a pharmaceutically acceptable salt of its 3-sulfate ester, estra-5(10),7-dien-3α-ol-17-one 3-glucuronide or a pharmaceutically acceptable salt thereof, estra-5(10),7-dien-3β-ol-17-one 3-alkali metal salt, and estra-5(10),7-dien-3α-ol-17-one 3-alkali metal salt are useful in replacement therapy in estrogen deficiency. The compounds of this invention are therefore useful in providing estrogen replacement therapy following ovariectomy or menopause, and in relieving symptoms related to estrogen deficiency, including vasomotor symptoms, such as hot flushes, and other menopausal related conditions, such as vaginal atrophy, vaginitis, and atrophic changes of the lower urinary tract which may cause increased urinary frequency, incontinence, and dysuria. The compounds of this invention are useful in preventing bone loss and in the inhibition or treatment of osteoporosis. The compounds of this invention are cardioprotective and they are useful in the treatment of atherosclerosis. These cardiovascular protective properties are of great importance when treating postmenopausal patients with estrogens to prevent osteoporosis and in the male when estrogen therapy is indicated. The compounds of this invention are also antioxidants, and are therefore useful in treating or inhibiting free radical induced disease states. Specific situations in which antioxidant therapy is indicated to be warranted are with cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke. Additionally, the compounds of this invention are useful in the suppression of lactation, and in the prophylaxis and treatment of mumps orchitis.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following provides the preparation of representative compounds of this invention. In particular the preparation of the compounds shown in Schemes I and II are described. The compound numbering used in these schemes is also used in the following examples.

### EXAMPLE 1

### Preparation of: Estra-5(10),7-dien-3β-ol-17-one (5B) Estra-5(10),7-dien-3β-ol-17-one 3-sulfate ester sodium salt (7)

### 17-Ethylenedioxy-3-methoxyestra-1,3,5(10),7-tetraene (1)

A suspension of equilin 3-methylether (5 g, 18 mmol), ethylene glycol (10 mL, 0.18 mol) and p-toluenesulfonic acid monohydrate (0.1 g, 0.53 mmol) in toluene (100 mL) was heated to reflux for 8h with azeotropic removal of water using a Dean-Stark apparatus. The reaction mixture was washed with 5% aqueous potassium bicarbonate (2 X 25 mL). The organic layer was separated, dried over anhydrous potassium carbonate and concentrated to give **1** as a white solid (5.8 g, 99 %).
¹H NMR (CDCl₃)
7.8-6.74 (m, 3H), 5.4 (br s, 1H), 3.88 (m, 4H), 3.74 (s, 3H), 0.75 (s, 3H)
¹³C NMR (CDCl₃)
157.94, 134.84, 130.72, 129.48, 119.94, 114.70, 113.13, 112.83, 65.82, 65.14, 55.56, 50.25, 48.32, 40.72, 34.86, 32.80, 32.56, 30.48, 14.63

### 17-Ethylenedioxy-3-methoxyestra-2,5(10),7-triene (2)

To a solution of the arene **1** (1.63 g, 5 mmol) in THF (25 mL) was condensed liquid ammonia (100 mL). Small pieces of freshly cut lithium wire (0.6 g, 86 mmol) were added over a period of 5 min at -50 °C. The deep blue solution was stirred at -33 °C for 30 min and ethanol (100%, 10 mL) was added over a period of 10 min. The ammonia was allowed to evaporate and water (20 mL) was added. The biphasic mixture was transferred to a separatory funnel and extracted with ether (3 X 30 mL). The ether extracts were combined, washed with brine, dried over potassium carbonate and concentrated to afford a shiny white solid which was washed with ether (4 X 20 mL) to give the enol ether **2** (1.2 g, 73%).
¹H NMR (CDCl₃)
5.27 (br s, 1H), 4.66 (br s, 1H), 3.91 (m, 4H), 3.56 (s, 3H), 0.74 (s, 3H)
¹³C NMR (CDCl₃)
152.86, 137.94, 126.96, 123.11, 119.97, 114.76, 90.98, 65.64, 65.04, 54.20, 49.90, 48.42, 42.50, 34.74, 33.68, 31.72, 29.31, 28.91, 20.45, 14.83

### 17-Ethylenedioxyestra-5(10),7-dien-3-one (3)

To a solution of the enol ether **2** (0.5 g, 1.52 mmol) in dichloromethane (30 mL), THF (20 mL) and methanol (20 mL) was added water (30 mL) followed by oxalic acid dihydrate (1.2 g, 9.5 mmol) and the resulting biphasic mixture was vigorously stirred for 8.5 h. The layers were separated and the aqueous layer washed with dichloromethane (2 X 20 mL). The organic layers were combined, dried over anhydrous potassium carbonate and concentrated to give the ketone **3** as a glassy solid (0.47 g, 98%).
¹H NMR (CDCl₃)
5.27 (br s, 1H), 3.89 (m, 4H), 0.73 (s, 3H)
¹³C NMR (CDCl₃)
212.21, 137.85, 129.95, 123.85, 119.76, 114.33, 65.61, 64.99, 49.72, 48.19, 44.10, 43.40, 39.56, 34.67, 31.86, 31.73, 29.26, 28.87, 20.34, 14.81

### 17-Ethylenedioxyestra-5(10).7-dien-3-ol (4)

To a well-stirred suspension of lithium aluminum hydride (0.3 g, 7.9 mmol) in diethyl ether (20 mL) at 0 °C under nitrogen was added a solution of the ketone **3** (0.47 g, 1.52 mmol) in ether (20 mL). After stirring for 1.5 h the reaction mixture was quenched by the sequential addition of water (0.3 mL), 15% aqueous sodium hydroxide (0.3 mL) and water (1 mL) and stirred for 30 min. The inorganic precipitate was filtered out and washed with ether (3 X 10 mL). The ether extracts and washings were combined and concentrated to afford a mixture (4:1) of the 3α- and 3β- alcohols **4** as a foam (0.47 g, quant).
¹H NMR (CDCl₃)
5.17 (br s, 1H), 3.98 (m, 0.2H), 3.88 (m, 0.8H), 3.8 (m, 4H), 0.66 (s, 0.6H), 0.65 (s, 2.4H)

### 3α-Hydroxyestra-5(10),7-dien-17-one (5A) and 3β-Hydroxyestra-5(10),7-dien-17-one (5B)

To a solution of the alcohol **4** (0.46 g, 1.45 mmol) in acetone (10 mL), THF (3 mL) and water (1 mL) was added p-toluene sulfonic acid monohydrate (0.1 g, 0.52 mmol). After stirring for 10 h the reaction mixture was diluted with ether (50 mL) and washed with aqueous 5% potassium bicarbonate (2 X 20 mL), brine (25 mL) and dried over anhydrous sodium sulfate. Concentration of the organic layer gave a mixture of the alcohols **5A** and **5B** as a waxy solid (0.45 g, quant).
HPLC (IB-SIL C18-BD, 8µ, 50 X 250 mm, 60% methanol in water, 205nm) separation of 0.35g of an α/β mixture of alcohols provided **5A** (0.145 g) and **5B** (0.081 g) in pure form.
**5A**
   ¹H NMR (CDCl₃)
   5.30 (br s, 1H), 3.86 (m, 1H), 0.69 (s, 3H)
   ¹³C NMR (CDCl₃)
   220.56, 136.33, 128.45, 123.89, 116.34, 68.07, 50.68, 50.12, 43.47, 39.64, 36.18, 32.58, 32.33, 32.27, 28.41, 27.66, 20.03, 14.14
**5B**
   ¹H NMR (CDCl₃)
   5.38 (br s, 1H), 4.08 (m, 1H), 0.76 (s, 3H)
   ¹³C NMR (CDCl₃)
   220.56, 136.35, 128.40, 122.99, 116.41, 66.59, 50.75, 50.12, 43.24, 38.84, 36.17, 32.41, 32.38, 30.80, 28.39, 24.32, 20.00, 14.14

### 3β-(3,5-Dinitrobenzoyloxy)estra-5(10),7-dien- 17-one (6)

To a suspension of the alcohols **5** (0.45 g, 1.6 mmol), 3,5-dinitrobenzoic acid (0.44 g, 2 mmol) and triphenylphosphine (0.52 g, 2 mmol) in toluene (20 mL) at 0 °C, under nitrogen, was added diethylazodicarboxylate (0.32 mL, 2 mmol) dropwise over a period of 2 min. The reaction mixture was allowed to warm to room temperature and stirred for 16 h. It was then heated to 55-58 °C for 4h and concentrated to dryness. The crude product was purified by flash chromatography, eluting with hexanes/ethyl acetate (7:3) to give the ester **6** as a yellow solid (0.13 g, 17.4 %).
¹H NMR (CDCl₃)
9.2-8.4 (m, 3H), 5.54 (m, 1H), 5.41 (br s, 1H), 0.80 (s, 3H)

### 3β-Hydroxyesta-5(10),7-dien-17-one (5B)

A solution of the ester **6** (0.13 g, 0.28 mmol) in THF (10 mL) and water (2 mL) was treated with potassium carbonate (0.02 g, 0.14 mmol) and stirred for 8h. Lithium hydroxide (0.02 g, 0.84 mmol) was added and stirring was continued an additional 30 min. The reaction mixture was diluted with ether (20 mL), washed with aqueous 5% potassium bicarbonate (2 X 20 mL) and dried over anhydrous sodium sulfate. Evaporation of solvents gave the alcohol **7** as a waxy solid ( 0.073 g, 96%) with a purple hue. This product was found to be contaminated with small amounts of 3β-hydroxyestra-5(10),6,8-trien-17-one **14**.
¹H NMR (CDCl₃) agrees with the sample obtained from the preparative HPLC separation of 5**A and 5B**.
GC/MS (silylated derivative) Analysis of **5B**: M/Z = 344; retention time 15.592 min.

### 3β-Hydroxyestra-5(10),7-dien-17-one, 3-sulfate ester sodium salt (7)

Crude alcohol **5B** (0.07 g, 0.25 mmol) was dissolved in THF (5 mL) and treated with triethylamine-sulfur trioxide complex (0.1 g, 0.55 mmol) for 48 h. The crystalline precipitate was isolated through filtration, dissolved in water (5 mL) and treated with 0.1 NaOH_{aq}. to pH 10. The aqueous solution was washed with ether (2 X 10 mL) and lyophilized to provide **7** as a fluffy solid (70 mg).
LC/MS (Negative Ion Electron Spray) Analysis of **7**: M/Z = 351; retention time = 31.54 mins.
¹H NMR (CDCl₃)
0.78 (1H), 1.30 (1H), 1.47 (td, 1H), 1.77 (1H), 1.80 (1H), 1.84 (1H), 1.88 (1H), 1.93 (1H), 2.02 (1H), 2.08 (1H), 2.19 (dd, 1H), 2.21 (1H), 2.27 (1H), 2.30 (1H), 2.40 (1H), 2.44 (1H), 2.48 (dd, 1H), 2.54 (bd, 1H), 2.63 (bd, 1H), 4.78 (m, 1H), 5.40 (bs, 1H).
¹³C NMR (CDCl₃)
14.2, 20.7, 25.0, 29.1, 29.2, 32.8, 33.2, 36.7, 36.9, 44.2, 51.0, 51.5, 75.3, 117.1, 123.6, 129.3, 137.3, 223.3.

### EXAMPLE 2

### Preparation of: Estra-5(10),7-dien-3β-ol-17-one (5B) Estra-5(10),7-dien-3α-ol-17-one (5A) Estra-5(10),7-dien-3α-ol-17-one 3-sulfate ester triethylammonium salt (15)

### 17β-Acetoxyestra-5(10),7-dien-3-one (9)

A solution of 17β-hydroxyestra-5(10), 7-dien-3-one (1.24 g, 4.5 mmol) in a mixture of pyridine (20 mL) and dichloromethane (20 mL) was treated with acetic anhydride (2.0 eq, 0.86 mL) and DMAP (0.1 eq, 10 mg, 0.08 mmol). The solution was stirred 16h at room temperature and then poured into water (50 mL). The layers were separated and the aqueous layer was extracted with ethyl acetate (3 x 50 mL). The organic layers were combined, washed with saturated aqueous NaCl solution (50 mL), dried over magnesium sulfate and concentrated under reduced pressure to give an oil that was purified by flash column chromatography on silica gel eluting with 20% EtOAc/hexane to give the acetate (1.03 g, 72%) as a white foam. A small portion was recrystallized from 10% Et₂O/hexane to afford white needles (mp 113-4 °C).
¹H NMR (400 MHz, CDCl₃)
δ 5.29 (1H, m), 4.77 (dd, J=6.15, 3.7Hz, 1H), 2.76 (m, 2H), 2.62 (m, 2H), 2.50 (m, 4H), 2.28 (m, 2H), 2.40 (s, 3H), 1.96 (m, 2H), 1.90 (m, 1H), 1.74 (m, 1H), 1.42 (dt, 13.2, 3.9Hz, 1H), 1.24 (dq, J=12, 3.9Hz, 1H), 0.69 (s, 3H). *m* / *z* (EI) 314 (M⁺).
Anal. (C₂₀H₂₆O₃) C, H, N: calcd C: 76.4; H, 8.34, N, 0.0; found C, 76.18, H, 8.20, N, 0.06.

### 17β-Acetoxyestra-5(10),7-dien-3-ol (10)

A solution of the ketone **9** (1.03 g, 3.28 mmol) in dry THF (20 mL) at 0 °C under nitrogen was treated, *via* syringe, with lithium tri-tert-butoxyaluminohydride (1.0 M, 1.5 eq, 4.92 mL, 4.92 mmol). After 2h the solution was poured into IN HCl (100 mL). The layers were separated and the aqueous layer was extracted with ether (3 x 50 mL). The organic layers were combined, washed with saturated aqueous NaCl solution (50 mL), dried over magnesium sulfate and concentrated under reduced pressure to give a white solid (1.08 g), R_{f} 0.3 (30% EtOAc/hexane), which was used without further purification.

### 17β-Acetoxy-3-(tert-butyldiphenylsilyl)oxyestra-5(10),7-diene (11)

To a solution of the alcohols **10** (1.08g, 3.42 mmol) in dichloromethane (20 mL) was added imidazole (1.6 eq, 0.37 g, 5.43 mmol) followed by *tert*-butyldiphenylsilylchloride (1.6 eq, 1.42 mL, 3.64 mmol). The mixture was stirred 16h at room temperature under nitrogen then poured into IN HCl (100 mL). The layers were separated and the aqueous layer was extracted with ether (3 x 50 mL). The organic layers were combined, washed with saturated aqueous NaCl solution (50 mL), dried over magnesium sulfate and concentrated under reduced pressure to give an oil (2.2 g), R_{f}0.55 (10% EtO Ac/hexane), which was used without further purification.

### 3-(tert-Butyldiphenylsilyl)oxy-17β-hydroxyestra-5(10),7-diene (12)

To the acetates **11** (2.2 g, 4.0 mmol) in a mixture of methanol (60 mL) and dichloromethane (10 mL) was added potassium carbonate (0.1 eq, 55 mg, 0.4 mmol). After stirring for 4h an additional portion of potassium carbonate (1.0 eq, 0.55 g, 4.0 mmol) was added. The mixture was stirred 16h and more potassium carbonate (1.0 eq, 0.55 g, 4.0 mmol) was added. After 24h water (100 mL) was added, the layers were separated and the aqueous layer was extracted with dichloromethane (3 x 100 mL). The organic layers were combined, washed with saturated aqueous NaCl solution (100 mL), dried over magnesium sulfate and concentrated under reduced pressure to give an oil that was purified by flash column chromatography on silica gel eluting with 20% EtOAc/hexane to give the alcohols **12** (1.34 g), R_{f}0.5 (30% EtOAc/hexane), as a white foam which was used directly.

### 3-(tert-Butyldiphenylsilyl)oxyestra-5(10,7-dien-17-one (13)

To the alcohols **12** (1.34 g, 2.6 mmol) in DMSO (20 mL) containing triethylamine (11 eq, 4.0 mL, 28.7 mmol) was added trimethylamine•sulfur trioxide complex (5.4 eq, 1.97 g, 14 mmol). After stirring for 16h under nitrogen water (100 mL) was added to the mixture and the aqueous phase was extracted with Et₂O (6 x 100 mL). The organic layers were combined and washed successively with IN aqueous HCl (100 mL), saturated aqueous sodium bicarbonate solution (100 mL) and saturated aqueous NaCl solution (100 mL). The organic layer was dried over magnesium sulfate and concentrated under reduced pressure to give an oil that was purified by flash column chromatography on silica gel eluting with 10% EtOAc/hexane to give the silyl protected alcohols **13** (1.34 g), R_{f} 0.5 (20% EtOAc/hexane), as a white foam which was used directly.

### Estra-5(10), 7-dien-3α-ol-17-one (5A) and Estra-5(10),7-dien-3β-ol-17-one (5B)

To a solution of the silyl protected alcohols **13** (1.16 g, 2.3 mmol) in THF (10 mL) at room temperature under nitrogen was added, *via* syringe, a solution of tetrabutylammonium fluoride (1.0 M, 1.3 eq, 2.9 mL, 3 mmol). After stirring for 24 h the solution was poured into water (100 mL) and the aqueous layer was extracted with ethyl acetate (3 x 100 mL). The organic layers were combined and washed successively with IN aqueous HCl (100 mL), saturated aqueous sodium bicarbonate solution (100 mL) and saturated aqueous NaCl solution (100 mL). The organic layer was dried over magnesium sulfate and concentrated under reduced pressure to give an oil that was purified by flash column chromatography on silica gel, eluting with 40% EtOAc/hexane to provide a white foam (0.56 g). Further purification by preparative HPLC (Primesphere, C₁₈-HC, 10µ, 50 x 20 mm; isocratic: 50/50 MeCN/H₂O; Flow 50 mL/min) gave-
**14**[*t*_{R}= 13.85 min, stereoisomers of the B-ring aromatic analog]
¹H NMR (300 MHz, CDCl₃)
δ 6.96 (brs, 2H) 4.15 (m, 1H), 2.97 (m, 2H), 2.95-2.50 (m, 6H), 2.50-2.25 (m, 2H), 2.05 (m, 2H), 1.85 (m, 3H), 0.77 and 0.75 (2 singlets, 2H).
*m* / *z* (ES-pos) 293 (M+Na⁺)
**5A**[*t*_{R}= 17.16 min] (330 mg), mp 139-42 °C.
¹H NMR (300 MHz, CDCl₃)
δ 5.37 (m, 1H), 3.93 (m, 1H), 2.61 (m, 2H), 2.48 (m, 2H), 2.45-2.10 (m, 5H), 2.10-1.80 (m, 7H), 1.7-1.4 (m, 2H), 1.26 (m, 2H), 0.76 (s, 3H).
*m* / *z* (ES-pos) 295 (M+Na⁺).
**5B** [*t*_{R}= 18. 68 m] (50 mg), mp 124-7 °C.
¹H NMR (300 MHz, CDCl₃)
δ 5.38 (m, 1H), 4.10 (m, 1H), 2.75-2.45 (m, 3H), 2.45-2.15 (m, 5H), 2.15-1.95 (m, 2H), 1.95-1.70 (m, 7H), 1.65-1.40 (m, 3H), 1.27 (m, 12H), 0.76 (s, 3H). *m* /*z* (ES-pos) 295 (M+Na⁺).

### Estra-5(10),7-dien-3α-ol-17-one, 3-sulfate ester triethylammonium salt (15)

To a solution of the alcohol **5A** (0.183 g, 0.67 mmol) in THF (2 mL) at room temperature was added pyridine-sulfur trioxide complex (1.3 eq, 0.14 g, 0.87 mmol). The mixture was stirred 4 days under nitrogen and then the THF was removed under reduced pressure. The solid residue was washed with ether (20 mL) and dissolved in a mixture of methanol (10 mL) and water (10 mL). Dowex-50 resin (700 mg) was added, the mixture was concentrated under reduced pressure and transferred to a sintered glass funnel. The resin was washed with methanol/water (1:1) until TLC analysis of the washings indicated all the product (ca. 200 mg) had been washed off. Further purification by HPLC (converted to the product to the triethylammonium salt during chromatography) (Primesphere, C₁₈-HC, 10µ, 50 x 250 mm, S#171320; 10/90 to 25/75 @ 5' to 50/50 @ 15' to 60/40 @ 23' to 65/35 @ 26.5' to 80/20 @ 36' to 90/10 @ 31'(MeOH /50 mM triethylammonium acetate (TEAA); pH=7); Flow 70.0 mL/min); 950PSI 850UV=214nm; 900PSI gave **15** [*t*_{R} = 31 min] (21 mg, 7%) as a gum..
¹H NMR (300 MHz, MeOH-*d*₄)
5.39 (s, 1H), 4.55 (m, 1H), 3.18 (q, J=7.3Hz, 7.6H), 2.61 (br s, 2H), 2.55-1.60 (m, 16H), 1.50 (m, 1H), 1.30 (t, J=7.3Hz, 12.5H), 0.76 (s, 3H). *m* /*z* (ES-neg) 351 (M-NH(C₂H₅)₃⁻).

## Claims

1. A compound which is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

2. The compound of claim 1 wherein the pharmaceutically acceptable salt of the 3-sulfate ester or 3-glucuronide is an alkali metal salt, alkaline earth metal salt, ammonium salt, alkylammonium salt containing 1-6 carbon atoms, or dialkylammonium salt containing 1-6 carbon atoms in each alkyl group, or trialkylammonium salt containing 1-6 carbon atoms in each alkyl group.

3. The compound of claim 2 which is the 3-sulfate ester of estra-5(10),7-dien-3β-ol-17-one.

4. Estra-5(10),7-dien-3β-ol-17-one 3-sulfate ester sodium salt.

5. A compound which is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

6. The compound of claim 5 wherein the pharmaceutically acceptable salt of the 3-sulfate ester or 3-glucuronide is an alkali metal salt, alkaline earth metal salt, ammonium salt, alkylammonium salt containing 1-6 carbon atoms, or dialkylammonium salt containing 1-6 carbon atoms in each alkyl group, or trialkylammonium salt containing 1-6 carbon atoms in each alkyl group.

7. The compound of claim 6 which is the 3-sulfate ester of estra-5(10),7-dien-3α-ol-17-one.

8. The compound of claim 5 which is estra-5(10),7-dien-3α-ol-17-one 3-sulfate ester triethylammonium salt.

9. Use of an antioxidant compound for the manufacture of a medicament for inhibiting or treating free radical induced disease states wherein the compound used is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

10. Use of a compound for the manufacture of a medicament for inhibiting endogenous free radical involvement in disease development of cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures, wherein the compound used is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

11. Use of an antioxidant compound for the manufacture of a medicament for inhibiting or treating free radical induced disease states wherein the compound used is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

12. Use of a compound for the manufacture of a medicament for inhibiting endogenous free radical involvement in disease development of cancers, central nervous system disorders, dimentias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures, wherein the compound used is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

13. Use of a compound for the manufacture of a medicament for providing estrogen replacement therapy or treating estrogen deficiency in a mammal in need thereof, wherein the compound used is an estrogenic amount of estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

14. Use of a compound for the manufacture of a medicament for providing estrogen replacement therapy or treating estrogen deficiency in a mammal in need thereof, wherein the compound used is an estrogenic amount of estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

15. Use of a compound for the manufacture of a medicament for treating vasomotor symptoms related to estrogen deficiency in a mammal in need thereof, wherein the compound used is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

16. The use of claim 15, wherein the vasomotor symptom is hot flushes.

17. Use of a compound for the manufacture of a medicament for treating vasomotor symptoms related to estrogen deficiency in a mammal in need thereof, wherein the compound used is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

18. The use of claim 17, wherein the vasomotor symptom is hot flushes.

19. Use of a compound for the manufacture of a medicament for treating or inhibiting osteoporosis in a mammal in need thereof wherein the compound used is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

20. Use of a compound for the manufacture of a medicament for treating or inhibiting osteoporosis in a mammal in need thereof wherein the compound used is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

21. Use of a compound for the manufacture of a medicament for treating or inhibiting atherosclerosis in a mammal in need thereof, wherein the compound used is estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof.

22. Use of a compound for the manufacture of a medicament for treating or inhibiting atherosclerosis in a mammal in need thereof, wherein the compound used is estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof.

23. A pharmaceutical composition which comprises estra-5(10),7-dien-3β-ol-17-one or a pharmaceutically acceptable salt of its 3-sulfate ester or estra-5(10),7-dien-3β-ol-17-one 3-glucuronide or a pharmaceutically acceptable salt thereof and a pharmaceutical carrier.

24. A pharmaceutical composition which comprises estra-5(10),7-dien-3α-ol-17-one or a pharmaceutically acceptable salt of its 3-sulfate ester or estra-5(10),7-dien-3α-ol-17-one 3-glucuronide or a pharmaceutically acceptable salt thereof and a pharmaceutical carrier.

25. A compound which is estra-5(10),7-dien-3β-ol-17-one 3-alkali metal salt.

26. A compound which is estra-5(10),7-dien-3α-ol-17-one 3-alkali metal salt.

## Patentansprüche

1. Verbindung, welche Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

2. Verbindung nach Anspruch 1, worin das pharmazeutisch annehmbare Salz des 3-Sulfatesters oder 3-Glucuronids ein Alkalimetallsalz, Erdalkalimetallsalz, Ammoniumsalz, Alkylammoniumsalz, welches 1-6 Kohlenstoffatome enthält, oder Dialkylammoniumsalz, welches 1-6 Kohlenstoffatome in jeder Alkylgruppe enthält, oder Trialkylammoniumsalz, welches 1-6 Kohlenstoffatome.in jeder Alkylgruppe enthält, ist.

3. Verbindung nach Anspruch 2, welche der 3-Sulfatester von Estra-5(10),7-dien-3β-ol-17-on ist.

4. Estra-5(10),7-dien-3β-ol-17-on-3-sulfatester-natriumsalz.

5. Verbindung, welche Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon ist.

6. Verbindung nach Anspruch 5, worin das pharmazeutisch annehmbare Salz des 3-Sulfatesters oder 3-Glucuronids ein Alkalimetallsalz, Erdalkalimetallsalz, Ammoniumsalz, Alkylammoniumsalz, welches 1-6 Kohlenstoffatome enthält, oder Dialkylammoniumsalz, welches 1-6 Kohlenstoffatome in jeder Alkylgruppe enthält, oder Trialkylammoniumsalz, welches 1-6 Kohlenstoffatome in jeder Alkylgruppe enthält ist.

7. Verbindung nach Anspruch 6, welche der 3-Sulfatester von Estra-5(10),7-dien-3α-ol-17-on ist.

8. Verbindung nach Anspruch 5, welche Estra-5(10),7-dien-3α-ol-17-on-3-sulfatester-triethylammoniumsalz ist.

9. Verwendung einer Antioxidans-Verbindung zur Herstellung eines Arzneimittels zum Hemmen oder Behandeln von durch freie Radikale verursachten Krankheitszuständen, wobei die verwendete Verbindung Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

10. Verwendung einer Verbindung zur Herstellung eines Arzneimittels zum Hemmen von endogener Einbeziehung von freien Radikalen in die Krankheitsentwicklung von Krebsen, Störungen des zentralen Nervensystems, Demenzen, Alzheimererkrankung, Knochenerkrankung, Alterung, Entzündungsstörungen, peripher-vaskulärer Krankheit, Rheumatoidarthritis, Autoimmunerkrankung, Atemnot, Emphysem, Verhinderung von Reperfusionsverletzung, viraler Hepatitis, chronisch aktiver Hepatitis, Tuberkulose, Psoriasis, systemischem Lupus erythematodes, Atemnotsyndrom des Erwachsenen, Trauma und Schlaganfall des zentralen Nervensystems oder Verletzung während Reperfusionsverfahren, wobei die verwendete Verbindung Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

11. Verwendung einer Antioxidans-Verbindung zur Herstellung eines Arzneimittels zum Hemmen oder Behandeln von durch freie Radikale verursachten Krankheitszuständen, wobei die verwendete Verbindung Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon ist.

12. Verwendung einer Verbindung zur Herstellung eines Arzneimittels zum Hemmen von endogener Einbeziehung von freien Radikalen in die Krankheitsentwicklung von Krebsen, Störungen des zentralen Nervensystems, Demenzen, Alzheimererkrankung, Knochenerkrankung, Alterung, Entzündungsstörungen, peripher-vaskulärer Krankheit, Rheumatoidarthritis, Autoimmunerkrankung, Atemnot, Emphysem, Verhinderung von Reperfusionsverletzung, viraler Hepatitis, chronisch aktiver Hepatitis, Tuberkulose, Psoriasis, systemischem Lupus erythematodes, Atemnotsyndrom des Erwachsenen, Trauma und Schlaganfall des zentralen Nervensystems oder Verletzung während Reperfusionsverfahren, wobei die verwendete Verbindung Estra-5(10),7-dien-3α-ol-17-on oder ein pharmazeutisch annehmbares Salz seines 3-Sulfatesters oder Estra-5 (10) , 7-dien-3α-ol-17-on-3-glucuronid oder ein pharmazeutisch annehmbares Salz davon ist.

13. Verwendung einer Verbindung zur Herstellung eines Arzneimittels zum Vorsehen von Östrogen-Ersatztherapie oder Behandeln von östrogenmangel in einem Säuger, der dessen bedarf, wobei die verwendete Verbindung eine östrogene Menge von Estra-5(10),7-dien-3β-ol-17-on oder eines pharmazeutisch annehmbaren Salzes seines 3-Sulfatesters oder Estra-5(10),7-dien-3β-ol-17-on-3-glucuronid oder eines pharmazeutisch annehmbaren Salzes davon ist.

14. Verwendung einer Verbindung zur Herstellung eines Arzneimittels zum Vorsehen von Östrogen-Ersatztherapie oder Behandeln von östrogenmangel in einem Säuger, der dessen bedarf, wobei die verwendete Verbindung eine östrogene Menge von Estra-5(10),7-dien-3α-ol-17-on oder eines pharmazeutisch annehmbaren Salzes seines 3-Sulfatesters oder Estra-5(10),7-dien-3α-ol-17-on-3-glucuronid oder eines pharmazeutisch annehmbaren Salzes davon ist.

15. Verwendung einer Verbindung zur Herstellung eines Arzneimittels zum Behandeln von vasomotorischen Symptomen in Verbindung mit Östrogenmangel in einem Säuger, der dessen bedarf, wobei die verwendete Verbindung Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

16. Verwendung nach Anspruch 15, wobei das vasomotorische Symptom Hitzewallungen ist.

17. Verwendung einer Verbindung zur Herstellung eines Arzneimittels zum Behandeln von vasomotorischen Symptomen in Verbindung mit Östrogenmangel in einem Säuger, der dessen bedarf, wobei die verwendete Verbindung Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon ist.

18. Verwendung nach Anspruch 17, wobei das vasomotorische Symptom Hitzewallungen ist.

19. Verwendung einer Verbindung zur Herstellung eines Arzneimittels zum Behandeln oder Hemmen von Osteoporose in einem Säuger, der dessen bedarf, wobei die verwendete Verbindung Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

20. Verwendung einer Verbindung zur Herstellung eines Arzneimittels zum Behandeln oder Hemmen von Osteoporose in einem Säuger, der dessen bedarf, wobei die verwendete Verbindung Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon ist.

21. Verwendung einer Verbindung zur Herstellung eines Arzneimittels zum Behandeln oder Hemmen von Atherosklerose in einem Säuger, der dessen bedarf, wobei die verwendete Verbindung Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon ist.

22. Verwendung einer Verbindung zur Herstellung eines Arzneimittels zum Behandeln oder Hemmen von Atherosklerose in einem Säuger, der dessen bedarf, wobei die verwendete Verbindung Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon ist.

23. Pharmazeutische Zusammensetzung, welche Estra-5(10),7-dien-3β-ol-17-on oder ein pharmazeutisch annehmbares Salz seines 3-Sulfatesters oder Estra-5(10),7-dien-3β-ol-17-on-3-glucuronid oder ein pharmazeutisch annehmbares Salz davon und einen pharmazeutischen Träger umfasst.

24. Pharmazeutische Zusammensetzung, welche Estra-5(10),7-dien-3α-ol-17-on oder ein pharmazeutisch annehmbares Salz seines 3-Sulfatesters oder Estra-5(10),7-dien-3α-ol-17-on-3-glucuronid oder ein pharmazeutisch annehmbares Salz davon und einen pharmazeutischen Träger umfasst.

25. Verbindung, welche Estra-5(10),7-dien-3β-ol-17-on-3-alkalimetallsalz ist.

26. Verbindung, welche Estra-5(10),7-dien-3α-ol-17-on-3-alkalimetallsalz ist.

## Revendications

1. Composé qui est l'oestra-5(10),7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

2. Composé suivant la revendication 1, dans lequel le sel pharmaceutiquement acceptable de l'ester 3-sulfate ou du 3-glucuronide est un sel de métal alcalin, un sel de métal alcalino-terreux, un sel d'ammonium, un sel d'alkylammonium contenant 1-6 atomes de carbone, ou un sel de dialkylammonium contenant 1-6 atomes de carbone dans chaque groupement alkyle, ou un sel de trialkylammonium contenant 1-6 atomes de carbone dans chaque groupement alkyle.

3. Composé suivant la revendication 2, qui est l'ester 3-sulfate de l'oestra-5(10), 7-dién-3β-ol-17-one.

4. Sel de sodium de l'ester 3-sulfate de l'oestra-5(10),7-dién-3β-ol-17-one.

5. Composé qui est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

6. Composé suivant la revendication 5, dans lequel le sel pharmaceutiquement acceptable de l'ester 3-sulfate ou du 3-glucuronide est un sel de métal alcalin, un sel de métal alcalino-terreux, un sel d'ammonium, un sel d'alkylammonium contenant 1-6 atomes de carbone, ou un sel de dialkylammonium contenant 1-6 atomes de carbone dans chaque groupement alkyle, ou un sel de trialkylammonium contenant 1-6 atomes de carbone dans chaque groupement alkyle.

7. Composé suivant la revendication 6, qui est l'ester 3-sulfate de l'oestra-5(10),7-dién-3α-ol-17-one.

8. Composé suivant la revendication 5, qui est le sel de triéthylammonium de l'ester 3-sulfate de l'oestra-5(10),7-dién-3α-ol-17-one.

9. Utilisation d'un composé antioxydant pour la fabrication d'un médicament pour inhiber ou traiter des états maladifs induits par les radicaux libres dans laquelle le composé utilisé est l'oestra-5(10),7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

10. Utilisation d'un composé pour la fabrication d'un médicament pour inhiber l'implication des radicaux libres endogènes dans le développement maladif de cancers, de troubles du système nerveux central, de démences, de la maladie d'Alzheimer, d'une maladie des os, du vieillissement, de troubles inflammatoires, d'une maladie vasculaire périphérique, de l'arthrite rhumatoïde, de maladies auto-immunes, d'une détresse respiratoire, d'emphysème, pour empêcher une lésion due à une reperfusion, une hépatite virale, une hépatite active chronique, la tuberculose, le psoriasis, le lupus érythémateux disséminé, un syndrome de détresse respiratoire chez l'adulte, un traumatisme du système nerveux central et un ictus ou une blessure pendant des procédures de reperfusion, dans laquelle le composé utilisé est l'oestra-5(10),7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

11. Utilisation d'un composé antioxydant pour la fabrication d'un médicament pour inhiber ou traiter des états maladifs induits par les radicaux libres dans laquelle le composé utilisé est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

12. Utilisation d'un composé pour la fabrication d'un médicament pour inhiber l'implication des radicaux libres endogènes dans le développement maladif de cancers, de troubles du système nerveux central, de démences, de la maladie d'Alzheimer, d'une maladie des os, du vieillissement, de troubles inflammatoires, d'une maladie vasculaire périphérique, de l'arthrite rhumatoïde, de maladies auto-immunes, d'une détresse respiratoire, d'emphysème, pour empêcher une lésion due à une reperfusion, une hépatite virale, une hépatite active chronique, la tuberculose, le psoriasis, le lupus érythémateux disséminé, un syndrome de détresse respiratoire chez l'adulte, un traumatisme du système nerveux central et un ictus ou une blessure pendant des procédures de reperfusion, dans laquelle le composé utilisé est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

13. Utilisation d'un composé pour la fabrication d'un médicament pour procurer une oestrogénothérapie de substitution ou pour traiter une déficience en oestrogènes chez un mammifère en ayant besoin, dans laquelle le composé utilisé est une quantité oestrogénique d'oestra-5(10),7-dién-3β-ol-17-one ou d'un sel pharmaceutiquement acceptable de son ester 3-sulfate ou de 3-glucuronide de l'oestra-5(10),7-dién-3β-ol-17-one ou d'un sel pharmaceutiquement acceptable de celui-ci.

14. Utilisation d'un composé pour la fabrication d'un médicament pour procurer une oestrogénothérapie de substitution ou pour traiter une déficience en oestrogène chez un mammifère en ayant besoin, dans laquelle le composé utilisé est une quantité oestrogénique d'oestra-5(10),7-dién-3α-ol-17-one ou d'un sel pharmaceutiquement acceptable de son ester 3-sulfate ou de 3-glucuronide de l'oestra-5(10),7-dién-3α-ol-17-one ou d'un sel pharmaceutiquement acceptable de celui-ci.

15. Utilisation d'un composé pour la fabrication d'un médicament pour le traitement de symptômes vasomoteurs associés à une carence en oestrogènes chez un mammifère en ayant besoin, dans laquelle le composé utilisé est l'oestra-5(10),7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

16. Utilisation suivant la revendication 15, dans laquelle le symptôme vasomoteur comprend des bouffées de chaleur.

17. Utilisation d'un composé pour la fabrication d'un médicament pour le traitement de symptômes vasomoteurs associés à une carence en oestrogènes chez un mammifère en ayant besoin, dans laquelle le composé utilisé est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

18. Utilisation suivant la revendication 17, dans laquelle le symptôme vasomoteur comprend des bouffées de chaleur.

19. Utilisation d'un composé pour la fabrication d'un médicament pour le traitement ou l'inhibition de l'ostéoporose chez un mammifère en ayant besoin, dans laquelle le composé utilisé est l'oestra-5(10),7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

20. Utilisation d'un composé pour la fabrication d'un médicament pour le traitement ou l'inhibition de l'ostéoporose chez un mammifère en ayant besoin, dans laquelle le composé utilisé est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

21. Utilisation d'un composé pour la fabrication d'un médicament pour le traitement ou l'inhibition de l'athérosclérose chez un mammifère en ayant besoin, dans laquelle le composé utilisé est l'oestra-5(10), 7-dién-3β-ol-17-one ou un sel pharmaceutiquement acceptable de son ester 3-sulfate ou le 3-glucuronide de l'oestra-5(10),7-dién-3β-ol-17-one ou un sel pharmaceutiquement acceptable de celui-ci.

22. Utilisation d'un composé pour la fabrication d'un médicament pour le traitement ou l'inhibition de l'athérosclérose chez un mammifère en ayant besoin, dans laquelle le composé utilisé est l'oestra-5(10),7-dién-3α-ol-17-one ou un sel pharmaceutiquement acceptable de son ester 3-sulfate ou le 3-glucuronide de l'oestra-5(10),7-dién-3α-ol-17-one ou un sel pharmaceutiquement acceptable de celui-ci.

23. Composition pharmaceutiquement acceptable qui comprend de l'oestra-5(10),7-dién-3β-ol-17-one ou un sel pharmaceutiquement acceptable de son ester 3-sulfate ou du 3-glucuronide de l'oestra-5 (10) , 7-dién-3β-ol-17-one ou un sel pharmaceutiquement acceptable de celui-ci et un vecteur pharmaceutique.

24. Composition pharmaceutiquement acceptable qui comprend de l'oestra-5(10),7-dién-3α-ol-17-one ou un sel pharmaceutiquement acceptable de son ester 3-sulfate ou du 3-glucuronide de l'oestra-5(10),7-dién-3α-ol-17-one ou un sel pharmaceutiquement acceptable de celui-ci et un vecteur pharmaceutique.

25. Composé qui est un sel de métal alcalin en 3 de l'oestra-5(10),7-dién-3β-ol-17-one.

26. Composé qui est un sel de métal alcalin en 3 de l'oestra-5(10),7-dién-3α-ol-17-one.
